# EUROPÄISCHE PATENTSCHRIFT

(11) **EP 0 468 192 B1**
(45) Veröffentlichungstag und Bekanntmachung des Hinweises auf die Patenterteilung: **11.09.1996**
(21) Anmeldenummer: 91109772.3
(22) Anmeldetag: 14.06.1991
(51) Int. Cl.: A61B 17/58

(54) **Osteosyntheseplatte**
Osteosynthetic plate
Plaque d'ostéosynthèse

(30) Priorität: 23.07.1990 CH 2435/90
(43) Veröffentlichungstag der Anmeldung: 29.01.1992
(73) Patentinhaber: Synthes AG, Chur, CH-7002 Chur (CH)
(72) Erfinder: Frigg, Robert, CH-7270 Davos-Platz (CH)
(74) Vertreter: Lusuardi, Werther Giovanni, Dr.

(56) Entgegenhaltungen:
- WO-A-89/09037
- DE-A- 2 705 154
- FR-A- 2 291 734
- US-A- 3 814 089

## Beschreibung

Die Erfindung bezieht sich auf eine Osteosyntheseplatte gemäss dem Oberbegriff des Anspruchs 1.

Bei der operativen Versorgung von Knochenbrüchen hat sich die sogenannte Verplattung etabliert. Die Form und Konstruktion von Knochenplatten variiert je nach Indikation und Anatomie des zu fixierenden Knochens.
Im Gegensatz zur sogenannten Marknagelung, ist das Ziel der Plattenosteosynthese eine möglichst genaue Reposition der einzelnen Knochenfragmente, sowie eine optimale Stabilität der Fraktur. Nur beim Erreichen dieser beiden Faktoren, kann eine primäre Frakturheilung erzielt werden. Diese wiederum schützt das Implantat gegen Übermüdung.
Im Laufe der letzten Jahre konnte, bei Schaftfrakturen, eine Tendenz von der Plattenosteosynthese weg zur Marknagelung beobachtet werden. Diese Tendenz hat vor allem zwei Gründe. Dank der Weiterentwicklung und Verbesserung von röntgenstrahlenbetriebenen Bildverstärkern, können Frakturen ohne direkten Sichtkontakt reponiert und genagelt werden. Der zweite Grund liegt in der biologischeren Vorgehensweise; d.h. die Frakturzone wird nicht mehr freigelegt, was zu einer erheblichen Verminderung der zirkulären Durchblutungsstörung beiträgt. Trotz der anfänglichen Begeisterung zeichnen sich jedoch auch die Nachteile der Marknagelung immer deutlicher ab. Neben der trickreichen und weichteilgefährdenden Eröffnung des Markraumes, wird auch die zentrale Durchblutung des Knochens auf seiner ganzen Länge nachhaltend zerstört.

Aus der FR-A-2291734 TORNIER ist eine Ellbogenprothese bekannt, welche einen verjüngten Plattenteil aufweist und welche mit einem zangenartigen Instrument der Knochenoberfläche angepasst werden kann.

Aus der WO89/09037 PAWLUK ist eine Knochenplatte bekannt, die ein verjüngtes Ende mit einer Abschrägung aufweist und asymmetrisch angeordnete Plattenlöcher aufweist.

Schliesslich ist aus der DE-A-2705154 BAUMANN ein lediglich als Zielinstrument dienender Zielbügel für einen im Markkanal eines Knochens einzuführenden Marknagel mit Querverriegelungslöchern. Dieses Zielinstrument dient allerdings nicht der Positionierung des Marknagels, der in üblicher Weise in den Markkanal eingeschlagen wird.

Hier will die Erfindung Abhilfe schaffen. Der Erfindung liegt die Aufgabe zugrunde, eine Osteosyntheseplatte zu schaffen, welche in kontrollierter Weise und unter möglichster Schonung des Knochens sowie der Weichteile, nur durch einen kleinen Schnitt von aussen auf dem Knochen positionierbar ist.

Die Erfindung löst die gestellte Aufgabe mit einer Osteosyntheseplatte, welche die Merkmale des Anspruchs 1 aufweist.

Grundsätzlich entspricht die erfindungsgemässe Osteosyntheseplatte einem Verriegelungsnagel, der aber markraumextern zu liegen kommt. Wie bei der Marknagelung liegt der Zugang frakturfern, kann jedoch gegenüber der Marknagelung, frei gewählt werden, da er nicht in der Knochenlängsachse liegen muss. Nach einer minimalen, frakturfernen Inzision, wird ein in der Dimension der Platte entsprechender Kanal durch die Weichteile bis auf die Knochenoberfläche präpariert. Die erfindungsgemässe Osteosyntheseplatte, welche nach Anbringung des Führungsgriffes bügeleisenförmig gestaltet ist, wird nun mit ihrer Spitze durch diesen Kanal, an der Knochenoberfläche entlang, über die Fraktur vorgeschoben. Je nach anatomischen Gegebenheiten, muss ein zusätzlicher Kanal entlang dem Knochen präpariert werden. Dieser Kanal kann grundsätzlich stumpf präpariert werden, eventuell auch arthroskopisch. Diese zusätzliche Präparation ist jedoch selten erforderlich. Gegenüber herkömmlichen Platten muss die erfindungsgemässe Osteosyntheseplatte nicht dem Knochen angepasst werden, da sie bedingt durch ihre Länge die Funktion der Frakturüberbrückung besitzt. Das heisst, die Fraktur muss nicht 100% anatomisch reponiert werden und es wird auch keine Kompression auf die Fraktur ausgeübt.

Nach erfolgter Plazierung der erfindungsgemässe Osteosyntheseplatte innerhalb des präparierten Kanals, wird sie an den Knochen geschraubt. Diese Verschraubung wird wie folgt durchgeführt:

Dem an der Platte montierten Führungsgriff kommt, neben der Führungsfunktion beim Einsetzen der Platte, auch die Funktion als Zielhilfe beim Setzen der Knochenschrauben zu. Die dafür vorgesehenen Bohrungen im Führungsgriff, entsprechen den Schraubenlöchern in der Platte. Das Bohren, das Messen der benötigten Schraubenlänge, eventuelles Gewindeschneiden und das Setzen der Schrauben erfolgt gezielt durch den Führungsgriff und durch eine Stichinzision der Weichteile.

Gemäss einer bevorzugten Ausführungsform der Erfindung sind die Plattenlöcher konisch ausgebildet und vorzugsweise mit einem Innengewinde versehen. Die zur Fixierung der Platten verwendeten Schrauben weisen dementsprechend einen konisch auslaufenden Kopf auf, vorzugsweise mit konischem Aussengewinde. Die Schrauben werden durch die Plattenlöcher hindurch in den Knochen eingedreht. Bei vollständigem Eindrehen der Schraube, verspannt sich der konische Schraubenkopf in der konischen Plattenbohrung, ein Effekt, der durch die vorzugsweise angebrachten Gewindegänge noch unterstützt wird. Dieses Verspannen ist dann wichtig, wenn die Schraube nur unikortikal eingesetzt werden soll und wenn die Platte nicht auf der Knochenoberfläche aufliegt. Bei dieser Art der Verschraubung ist der Winkel zwischen der Platte und den Schrauben fixiert. Bei einer herkömmlichen Plattenosteosynthese bedarf es hingegen der sogenannten Platten-Knochenreibung, um eine Fraktur zu fixieren.
Die konische Schraubenverbindung hat zudem noch den Vorteil, dass sich die Gewindegänge, bei festem Anziehen, ineinander verkeilen. Dieses Verkeilen vermindert die Gefahr einer unbeabsichtigten Lockerung der rigiden Platten/Schrauben-Verbindung durch zyklische Belastungen.
Die rigide Platten/Schrauben-Verbindung lässt sich auch mittels Schrauben erreichen, welche über einen expandierbaren Kopf verfügen, wie beispielsweise in der WO 88/03781 offenbart.

Die bügeleisenförmige Gestalt der Platte wird vorzugsweise derart realisiert, dass die Plattenunterseite , einschliesslich der Plattenspitze, eine Gerade bildet. Die Plattenoberseite anderseits verläuft schlittenförmig nach unten zu einer Spitze. Vorzugsweise sollten sich auch die seitlichen Plattenwände leicht zu einer Spitze verjüngen. Diese Form bewirkt, dass sich die Platte beim Vorwärtsschieben über dem Knochen sich letzterem automatisch nähert, d.h. sie kann kontrolliert dem Knochen entlang vorwärts geschoben werden.
Alternativ dazu kann die Plattenunterseite, ebenso wie die Oberseite, leicht schlittenförmig ausgebildet sein. Die Schlittenspitze befindet sich bei dieser Ausführungsform annähernd in der Mittelachse der Platte. Die so ausgebildete Plattenspitze kann die Weichteile stumpf durchstossen, besitzt jedoch nicht den Trend, nur der Knochenoberfläche zu folgen, da sich die in den Weg stellenden Weichteile gleichmässig nach oben und unten verdrängt werden. Diese Ausführungsform eignet sich somit für diejenigen Fälle bei denen ohnehin (arthroskopisch) ein Kanal für die Einführung der Platte geformt werden muss und erlaubt es, sehr nahe der Plattenspitze eine Verankerungsschraube zu setzen, was bei einer kufenförmigen Spitze nicht möglich ist.

Die durch die Erfindung erreichten Vorteile sind im wesentlichen die folgenden:
- Die Osteosyntheseplatte kann als Überbrückungsplatte verwendet werden, so dass die Fraktur nicht 100%ig reponiert werden muss.
- Die Osteosyntheseplatte muss nicht an die Anatomie des zu behandelnden Knochens angepasst werden.
- Die Implantation erfolgt durch eine minimale, frakturferne Inzision. Eine zusätzliche Störung der Biologie rund um die Fraktur wird dadurch minimalisiert. Der kosmetische Vorteil einer mehrfach kürzeren Narbe ist erheblich.
- Einfaches, kontrolliertes Einführen der bügeleisenförmigen Platte mittels des Führungsgriffes.
- Das Bohren, Gewindeschneiden, Längenmessen und Setzen der Schrauben, kann gezielt durch den Führungsgriff hindurch erfolgen.
- Durch die feste Verankerung der Schraubenköpfe in den Plattenbohrungen kann die Platte von der Knochenoberfläche abstehen, was zur Aufrechterhaltung der Knochendurchblutung ausserordentlich wichtig ist.
- Da die Verankerung der Schrauben nur in der nahen Kortikalis nötig ist, kann das Schraubensortiment auf ca. drei Schrauben, pro Systemdimension reduziert werden.
- Die unikortikal verwendeten Schrauben können eine selbstschneidende Spitze besitzen, da diese in den Markraum zu liegen kommen. Eine Weichteilgefährdung durch eine schneidende Schraubenspitze ist nicht vorhanden.

Ein Ausführungsbeispiel der Erfindung, welches zugleich das Funktionsprinzip erläutert, ist in der Zeichnung dargestellt und wird im folgenden näher beschrieben.
Fig. 1 stellt einen Längsquerschnitt durch die erfindungsgemässe Osteosyntheseplatte mit daran befestigter Einführungs- und Zielvorrichtung dar;
Fig. 2 stellt einen Längsquerschnitt durch eine tibial implantierte erfindungsgemässe Osteosyntheseplatte mit daran befestigter Einführungs- und Zielvorrichtung dar; und
Fig. 3 stellt eine Aufsicht auf eine femoral applizierte erfindungsgemässe Osteosyntheseplatte mit bereits entfernter Einführungs- und Zielvorrichtung dar.

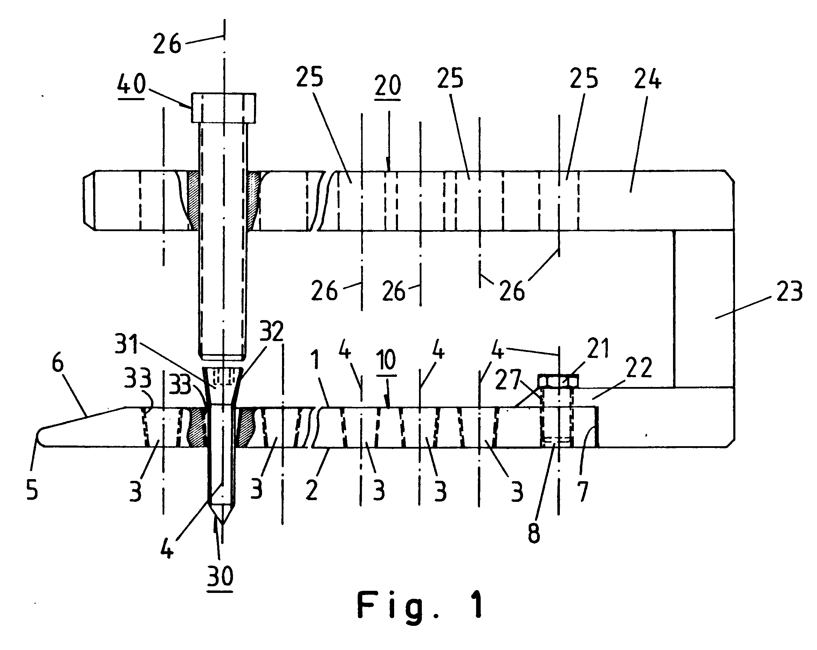

Die in Fig. 1 gezeigte Osteosyntheseplatte 10 weist im wesentlichen eine Oberseite 1, eine Unterseite 2, mehrere die Oberseite 1 mit der Unterseite 2 verbindende Bohrungen 3 zur Aufnahme von Knochenschrauben 30, sowie zwei Plattenenden 5 und 7 auf. Das eine Plattenende 5 weist - wie die Spitze eines Bügeleisens - eine Abschrägung 6 an der Oberseite 1 der Platte 10 auf, derart, dass bei der Einführung der Platte 10 durch die Weichteile ein möglichst geringer Widerstand und damit eine minimale Verletzung auftritt. Zu diesem Zweck können sich auch die seitlichen Plattenwände 9 leicht zu einer Spitze verjüngen. Das andere Plattenende 7 weist ein Kupplungsmittel in Form einer zylindrischen Gewindebohrung 8 auf in welche mittels einer Befestigungsschraube 21 eine Einführungs- und Zielvorrichtung 20 in lösbarer Weise fixiert werden kann.

Die Bohrungen 3 sind als sich gegen die Unterseite 2 hin verjüngende Koni mit einem Innengewinde 33 ausgebildet, welches mit dem Aussengewinde 32 der aufzunehmenden Knochenschrauben 30 korrespondiert. Dadurch lässt sich eine absolut rigide Verbindung zwischen Platte 10 und Schrauben 30 erreichen, was von ausschlaggebender Bedeutung ist. Der Konuswinkel liegt vorteilhafterweise zwischen 2,0° bis 4,0°, vorzugsweise zwischen 2,5° bis 3,5° und im speziellen bei 3°.

Die bügeleisenförmige Einführungs- und Zielvorrichtung 20 weist an ihrem zur Kupplung mit der Platte 10 bestimmten Ende 22 eine zylindrische Bohrung 27 auf, durch welche die Befestigungsschraube 21 geführt werden kann um die Einführungs- und und Zielvorrichtung 20 temporär an der Platte 10 zu befestigen. An das Ende 22 schliesst sich ein rechtwinklig angeordnetes Abstandstück 23 an, mit dem, parallel zur Platte 10 verlaufenden, eigentlichen Führungsgriff 24. Im Führungsgriff 24 der Einführungs- und Zielvorrichtung 20 sind zylindrische Bohrungen 25 angebracht, deren Achsen 26 genau mit den Achsen 4 der Platten-Bohrungen 3 übereinstimmen. Dadurch ist es möglich, mittels geeigneter Instrumente 40 (Bohrer, Längenmesser, Setzinstrumente u.s.w.), durch die Bohrungen 25 hindurch die koaxialen Schrauben 30 ohne Röntgengerät schnell und präzise mit der Platte 10 und im Kochen zu fixieren. Auch das Ankoppeln und Entkoppeln der Einführungs- und Zielvorrichtung 20 mittels der Befestigungsschraube 21 erfolgt durch die entsprechende Bohrung 25 im Führungsgriff 24 hindurch, welche mit der Bohrung 8 der Platte 10 fluchtet.

Die Dimensionen der Platte 10 richten sich weitgehend nach dem zu fixierenden Knochen, bzw. der zu versorgenden Fraktur. Für eine Anwendung an der Tibia beträgt die Plattenbreite etwa 12 mm, die Plattenhöhe etwa 4 mm und die Plattenlänge zwischen 200 und 300 mm. Bei einer Anwendung am Femur würden sich diese Dimensionen etwas vergrössern.
Das Abstandsstück 23 weist bei einer tibialen Anwendung eine Länge von etwa 50 mm, für eine femorale Anwendung eine Länge von etwa 100 mm auf.

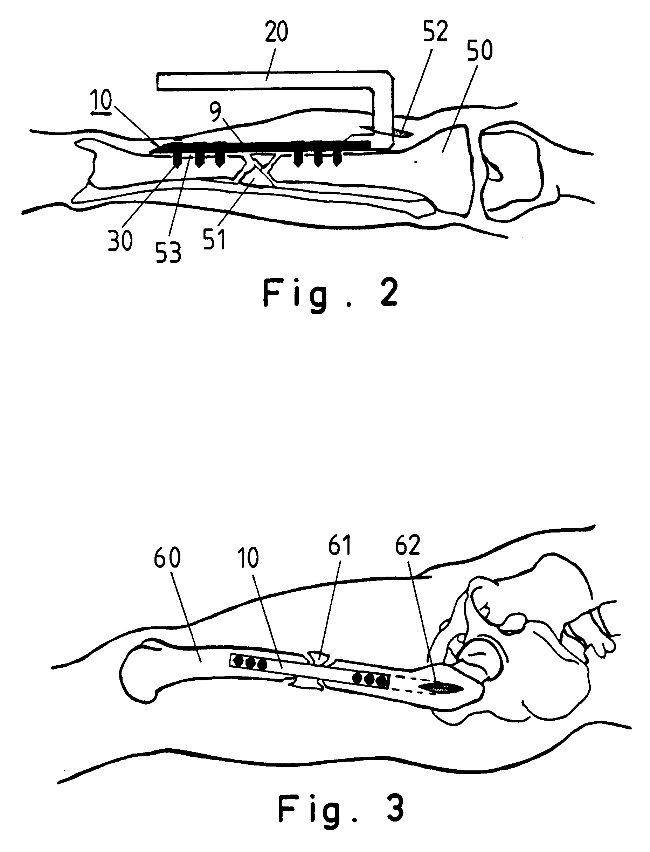

In Fig. 2 ist der Zustand einer solcherart zur Überbrückung einer Fraktur 51 der Tibia 50 implantierten Platte 10 dargestellt, an welcher die durch die Inzision 52 nach aussen ragende Einführungs- und Zielvorrichtung 20 noch befestigt ist. Die Platte 10 ist mit je drei distal und proximal von der Fraktur 51 angeordneten Schrauben 30 am Knochen befestigt, derart dass zwischen Knochen 50 und Platte 10 ein Zwischenraum 53 verbleibt, welcher die Revaskularisierung ermöglicht.

In Fig. 3 ist der Zustand einer analogen, zur Überbrückung einer Fraktur 61 des Femur 60 implantierten Platte 10 dargestellt. Die dazu verwendete (nicht dargestellte) Einführungs- und Zielvorrichtung ist hier bereits durch die Inzision 62 hindurch entfernt worden. Gleich wie in Fig. 2 dargestellt, werden bei dieser Operationstechnik die Einzelfragmente 61 nicht per se fixiert sondern durch die Platte 10 überbrückt.

## Patentansprüche

1. Osteosyntheseplatte (10) mit einer Oberseite (1), einer Unterseite (2), mehreren die Oberseite (1) mit der Unterseite (2) verbindenden Bohrungen (3) zur Aufnahme von Knochenschrauben (30), ein vorderes und ein hinteres Plattenende (5,7), sowie zwei seitlichen Plattenwänden (9), dadurch gekennzeichnet, dass
A) das vordere Plattenende (5) eine die Einführung der Osteosyntheseplatte (10) durch die Weichteile hindurch gestattende Verjüngung aufweist, und
B) das hintere Plattenende (7) temporär rigide, lösbare Kupplungsmittel (8) für einen parallel zur Osteosyntheseplatte (10) verlaufenden Führungsgriff (24) aufweist, der als Einführungs- und Positionierungsvorrichtung ausserhalb des Körpers dient.

2. Osteosyntheseplatte nach Anspruch 1, dadurch gekennzeichnet, dass die Verjüngung des vorderen Plattenendes (5) in Form einer Abschrägung (6) der Oberseite (1) realisiert ist.

3. Osteosyntheseplatte nach Anspruch 2, dadurch gekennzeichnet, dass die seitlichen Plattenwände (9) am vorderen Plattenende (5) zusammenlaufen, vorzugsweise unter Bildung einer Schneide oder Spitze.

4. Osteosyntheseplatte nach Anspruch 2 oder 3, dadurch gekennzeichnet, dass die Unterseite (2) am vorderen Plattenende (5) eine zusätzliche Abschrägung aufweist.

5. Osteosyntheseplatte nach einem der Ansprüche 1 - 4, dadurch gekennzeichnet, dass die Bohrungen (3) als sich gegen die Unterseite (2) hin verjüngende Koni ausgebildet sind, welche vorzugsweise ein Gewinde (33) aufweisen.

6. Osteosyntheseplatte nach Anspruch 5, dadurch gekennzeichnet, dass die Bohrungen (3) einen Konuswinkel von 2,0° bis 4,0°, vorzugsweise von 2,5° bis 3,5° aufweisen.

7. Osteosyntheseplatte nach einem der Ansprüche 1 - 6, dadurch gekennzeichnet, dass der Führungsgriff (24) als Zielvorrichtung für die von ausserhalb des Körpers einzusetzenden Knochenschrauben (30) dient.

8. Osteosyntheseplatte nach Anspruch 7, dadurch gekennzeichnet, dass im Führungsgriff (24), vorzugsweise zylindrische, Bohrungen (25) angebracht sind, deren Achsen (26) genau mit den Achsen (4) der Platten-Bohrungen (3) übereinstimmen.

## Claims

1. An osteosynthetic plate (10) having an upper surface (1), a lower surface (2), a plurality of holes (3) connecting the upper surface (1) with the lower surface (2) and adapted to receive bone screws (30), said plate (10) having an anterior plate end (5), a posterior plate end (7) and two side walls (9),
**characterized in that**
A) said anterior plate end (5) is provided with a taper facilitating the insertion of said osteosynthetic plate (10) through the soft tissues; and
B) said posterior plate end (7) is provided with temporary rigid engaging and disengaging coupling means (8) for a guide handle (24) running parallel to said osteosynthetic plate (10) and which serves as inserting and positioning device outside the body.

2. An osteosynthetic plate (10) according to claim 1, characterized in that the taper of the anterior plate end (5) is realised in the form of a bevel (6) at the upper surface (1).

3. An osteosynthetic plate (10) according to claim 2, characterized in that the side walls (9) taper inwardly at the anterior plate end (5), thereby preferably forming a cutting edge or a point.

4. An osteosynthetic plate (10) according to claim 2 or 3, characterized in that the lower surface (2) is provided with an additional bevel at the anterior plate end (5).

5. An osteosynthetic plate (10) according to one of the claims 1 - 4, characterized in that the holes (3) are conically shaped, tapering toward the lower surface (2), and are provided preferably with a thread (33).

6. An osteosynthetic plate (10) according to claim 5, characterized in that the holes (3) have a conical angle of from 2.0° to 4.0°, preferably from 2.5° to 3.5°.

7. An osteosynthetic plate (10) according to one of the claims 1 - 6, characterized in that the guide handle (24) serves as a guiding device for the bone screws (30) to be inserted from outside the body.

8. An osteosynthetic plate (10) according to claim 7, characterized in that holes (25), preferably of cylindrical shape, are provided in the guide handle (24), the axes (26) of said holes (25) coinciding exactly with the axes (4) of the holes (3) in the plate (10).

## Revendications

1. Plaque d'ostéosynthèse (10), comportant une face supérieure (1), une face inférieure (2), plusieurs alésages (3) reliant la face supérieure (1) à la face inférieure (2), pour la réception de vis osseuses (30), une extrémité avant et une extrémité arrière de plaque (5, 7), ainsi que deux parois latérales de plaque (9),
caractérisée en ce que
A) l'extrémité avant de la plaque (5) présente un rétrécissement permettant l'insertion de la plaque d'ostéosynthèse (10) à travers les tissus mous, et
B) l'extrémité arrière de la plaque (7) présente des moyens d'accouplement (8) temporaires, rigides et libérables, pour une poignée de guidage (24) s'étendant parallèlement à la plaque d'ostéosynthèse (10), et qui sert de dispositif d'insertion et de positionnement externe au corps.

2. Plaque d'ostéosynthèse selon la revendication 1, caractérisée en ce que le rétrécissement de l'extrémité avant de la plaque (5) est réalisé sous la forme d'un chanfreinage (6) de la face supérieure (1).

3. Plaque d'ostéosynthèse selon la revendication 2, caractérisée en ce que les parois latérales de la plaque (9) se rejoignent à l'extrémité avant de la plaque (5), de préférence en formant un tranchant ou une pointe.

4. Plaque d'ostéosynthèse selon la revendication 2 ou 3, caractérisée en ce que la face inférieure (2) présente à l'extrémité avant de la plaque (5) un chanfrein supplémentaire.

5. Plaque d'ostéosynthèse selon l'une des revendications 1-4, caractérisée en ce que les alésages (3) sont de forme conique se rétrécissant vers la face inférieure (2), et qui présentent de préférence un filet (33).

6. Plaque d'ostéosynthèse selon la revendication 5, caractérisée en ce que les alésages (3) présentent un angle de cône de 2,0° à 4,0°, de préférence de 2,5° à 3,5°.

7. Plaque d'ostéosynthèse selon l'une des revendications 1-6, caractérisée en ce que la poignée de guidage (24) sert de dispositif de visée pour les vis osseuses (30) à insérer depuis l'extérieur du corps.

8. Plaque d'ostéosynthèse selon la revendication 7, caractérisée en ce que dans la poignée de guidage (24) sont pratiqués des alésages (25) de préférence cylindriques, dont les axes (26) correspondent avec précision aux axes (4) des alésages (3) de la plaque.
